# EUROPEAN PATENT APPLICATION

(11) **EP 4 814 975 A1**
(43) Date of publication of application: **30.09.2026**
(21) Application number: 25305432.4
(22) Date of filing: 25.03.2025
(51) Int. Cl.: G16H 10/60, G06F 18/00, G16H 50/70

(54) **METHOD AND SYSTEM FOR IDENTIFYING A PATIENT BASED ON AN ECG SIGNAL**

(71) Applicant: Koninklijke Philips N.V., 5656 AE Eindhoven (NL)
(72) Inventor: DUPONT, Rémi, 5656 Eindhoven (NL); SHRESHTHA, Kumar, 5656 Eindhoven (NL); PEZZOTTI, Nicola, 5656 Eindhoven (NL); KHELDOUNI, Amine, 5656 Eindhoven (NL); CHOFFIN, Benoît, 5656 Eindhoven (NL); REBENA, Nicolas, 5656 Eindhoven (NL)
(74) Representative: Philips Intellectual Property & Standards

(57) **Abstract**

The invention relates to a system for identifying a patient in a patient database based on an ECG signal. The system comprising an input interface configured to receive an electrocardiogram ECG signal of the patient; encode the ECG signal using an encoder so as to obtain a current vector embedding; maintain a vector database comprising previous vector embeddings of ECG signals of multiple patients acquired over time; compare the current vector embedding with the previous vector embeddings; and identify the patient based on one or more previous vector embeddings that are the closest to the current vector embedding.

## Description

### FIELD OF THE INVENTION

This invention relates to the field of electrocardiogram ECG measurements and more specifically to a method and system for identifying a patient in a patient database based on an ECG signal.

### BACKGROUND OF THE INVENTION

ECGs are a diagnostic modality that is quite compelling as it is relatively easy to acquire signal without expensive devices or lengthy acquisition procedures. The resulting signals can tell a lot about the cardiac status of the patient, making it the ideal entry point of all cardiovascular disease CVD care pathways.

However, dealing with large database of ECGs can be difficult due to an overburden of data insertion in the database, making it difficult and time consuming to enter patient information for each ECG. Moreover, errors in insertion can lead to a disconnected database and an inability to track the patient.

### SUMMARY OF THE INVENTION

It is an aim of the invention to provide a method and system, which facilitate the identification of a patient.

The invention is defined by the appended claims.

According to examples in accordance with an aspect of the invention, there is provided a method for identifying a patient in a patient database based on an ECG signal.

The method comprises the steps of:
receiving an electrocardiogram ECG signal of the patient;
encoding the ECG signal using an encoder so as to obtain a current vector embedding;
maintaining a vector database comprising previous vector embeddings of ECG signals of multiple patients acquired over time;
comparing the current vector embedding with the previous vector embeddings; and
identifying the patient based on one or more previous vector embeddings that are the closest to the current vector embedding.

In an embodiment of the invention, the patient is identified by minimizing the Euclidian distance between the current and previous embedding vectors.

In another embodiment of the invention, the recorded ECG signal is a one-dimensional signal.

According to another aspect of the invention, there is provided a computer program comprising code means for implementing the method when said program is run on a processing system.

According to another aspect of the invention, there is provided a system for identifying a patient in a patient database based on an ECG signal.

The system comprises an input interface configured to:
receive an electrocardiogram ECG signal of the patient;
encode the ECG signal using an encoder so as to obtain a current vector embedding;
maintain a vector database comprising previous vector embeddings of ECG signals of multiple patients acquired over time;
compare the current vector embedding with the previous vector embeddings; and
identify the patient based on one or more previous vector embeddings that are the closest to the current vector embedding.

The system is advantageously included in a monitoring system comprising a cardiovascular monitor.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a schematical overview of the proposed method in accordance with an embodiment of the invention;
Fig. 2 shows the visualization of the vector embedding of multiple patients; and
Fig. 3 shows an example of an application combining distance from vector embedding and from patient information to identify typographic errors.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

While new health informatic IT infrastructure offers a more efficient pipeline to store and analyse ECGs, it requires to enter patient information each time an ECG of this patient is recorded. As any manual step, it can lead to errors within the patient data. This can lead to two patients entered in the database instead of a single one, making it difficult to group ECGs from the same patient, therefore having a global view of its situation. It is a purpose of this invention to propose a method and system that leverage advances of machine learning in the recent years in conjunction with the reduction of the computational cost to find ECGs closest to a just recorded ECG signal so as to identify ECGs from the same patient. Such a method and system can also overcome misdiagnosis since having all ECGs available for a same patient provides the practitioner with more complete information.

The system and method according to the invention propose a new health IT infrastructure adapted to store a high number of ECGs of multiple patients coming from different sources. The health IT infrastructure is designed to analyse the ECG data using an embedding algorithm to generate vector embeddings representative of the ECG data.

Thus, a new health IT infrastructure is proposed with vector databases of ECG vector embeddings (which can be extended to other complex data modalities) for each patient that facilitates matching of a new patient with her/his previous ECGs.

The invention makes it possible to get new patient information related to these closest vectors and compare it to patient information entered by the practitioner. If some patient information is very similar to the ones entered by the practitioner, it can be confirmed that this is the same patient information. The practitioner can correct any of the patient data if needed such as, for instance correct spelling in first name, last name, date of birth, etc.

Alternatively, the invention makes it possible for the practitioner not to enter patient information. Instead, patient information are prefilled with the ones from the closest ECG vector embedding. The practitioner can then either confirm or correct the automatically filled values.

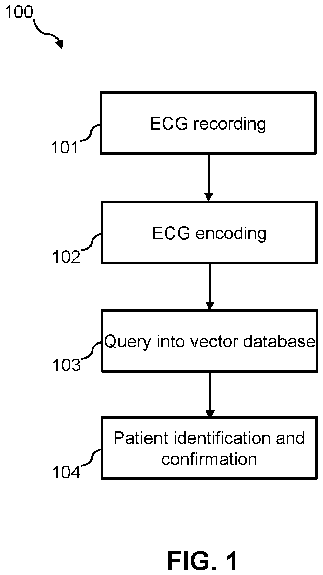

Fig. 1 is a schematical overview of the method in accordance with an embodiment of the invention.

According to this embodiment, the method includes a first step 101 during which an ECG signal is recorded in the electronic health record EHR during an inpatient regular exam or an outpatient visit. In more details, a first ECG signal is recorded for a patient and patient information such as family name, first name, birth date and sex are recorded at the same time. The recorded ECG signal is preferably a one-dimensional signal and not only a part of it. A few weeks or months later, a practitioner takes another ECG of the same patient for example in the emergency care services of an hospital.

In a second step 102, the recorded ECG signal is converted into a vector embedding using an ECG encoder. The encoder is a neural network, which typically includes a vision transformer that is trained in a self-supervised manner to detect features of the ECG such that the resulting vector embedding is close for ECGs that are similar while far apart for the ones that are dissimilar. The invention is not limited to this neural network and other deep-neural network architecture and algorithm may be used for this task.

In a third step 103, the ECG embedding is used to query into a vector database of patients from the same hospital, or from other hospital(s). It finds amongst the vector database one or more ECG vector embeddings that are the closest to the query patient using a Hierarchical Navigable Small Worlds HNSW algorithm for instance. For that purpose, a similarity score is computed based a similarity measure such as the cosine similarity, the Manhattan distance L1 or the Euclidian distance L2. The invention is not limited to these similarity measures and other measures can be used.

In a last step 104, the matched patients are then ranked based on their vector embeddings to the query patient and if the similarity score is below a certain threshold for a given patient, it is derived that those ECGs are coming from the same patient. This is the case for example if the cosine similarity is close to 1 or the Euclidian close to 0. The practitioner is then able to identify the current patient and to correct her / his patient data if needed.

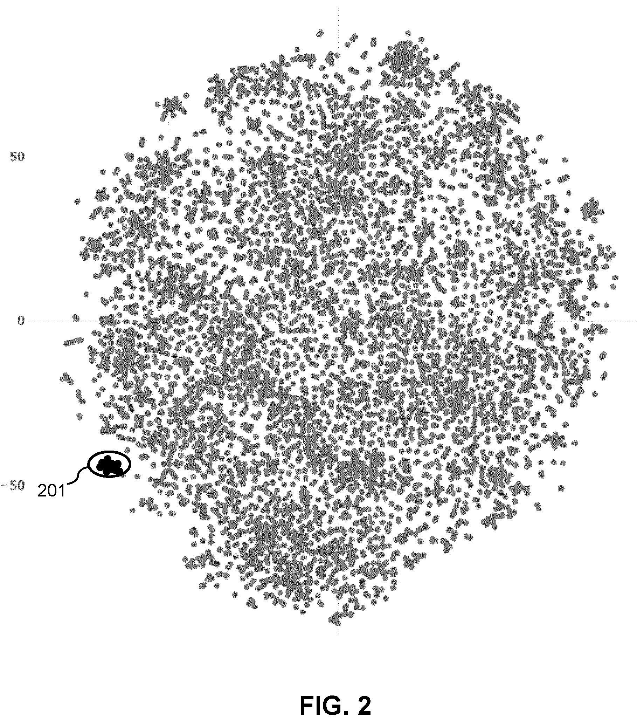

Fig. 2 shows the visualization of the vector embedding of multiple patients. The visualization is based on the t-SNE algorithm. This algorithm is a dimensionality reduction algorithm which is often used for visualization. It learns a mapping from a set of high-dimensional vectors to a space with a smaller number of dimensions (usually 2).

Fig. 2 demonstrates the ability of the method in accordance with the invention to successfully group multiple ECGs from the same patient together using ECG vector embeddings. The darkest points 201 correspond to the same patient.

Another application of this invention is to correct data entry errors in patient information using both distance from the patient information itself and distances from the embedding vectors.

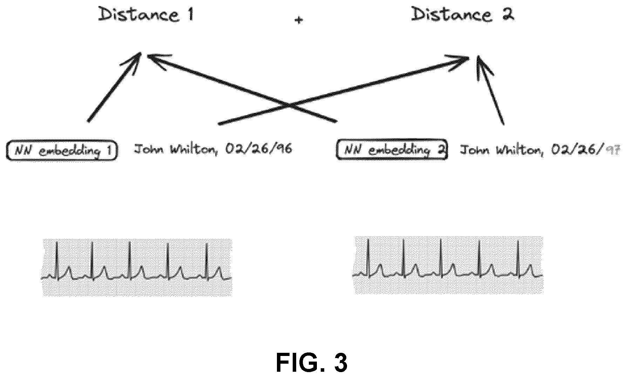

Fig. 3 shows an example of such an application combining distance from vector embedding (neural network embedding) and from patient information to identify typographic errors. Damerau-Levenshtein distance is used for example in distance 2, and distance 1 is a numerical vector distance sur as cosine similarity for instance. In this particular example, it is identified that the year of birth has to be corrected.

The above-described method and system may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for identifying a patient in a patient database based on an ECG signal, the method comprising the steps of:
receiving an electrocardiogram ECG signal of the patient;
encoding the ECG signal using an encoder so as to obtain a current vector embedding;
maintaining a vector database comprising previous vector embeddings of ECG signals of multiple patients acquired over time;
comparing the current vector embedding with the previous vector embeddings; and
identifying the patient based on one or more previous vector embeddings that are the closest to the current vector embedding.

2. The method of claim 1, wherein the patient is identified by minimizing the Euclidian distance between the current and previous embedding vectors.

3. The method of claim 1, wherein the patient is identified by minimizing the Manhattan distance between the current and previous embedding vectors.

4. The method of claim 1, wherein the patient is identified by determining the current and previous embedding vectors that have the highest cosine similarity.

5. The method of any preceding claim, wherein the encoder is a neural network, which includes a vision transformer that is trained in a self-supervised manner.

6. The method of any preceding claim, wherein the comparing step is based on a Hierarchical Navigable Small Worlds HNSW algorithm.

7. The method of any preceding claim, wherein the recorded ECG signal is a one-dimensional signal.

8. The method of any preceding claim further comprising the steps of:
entering patient information before acquiring the ECG signal; and
confirming that patient information are correct after that the patient has been identified.

9. A computer program comprising code means for implementing the method of any preceding claim when said program is run on a processing system.

10. A system for identifying a patient in a patient database based on an ECG signal, the system comprising an input interface configured to:
receive an electrocardiogram ECG signal of the patient;
encode the ECG signal using an encoder so as to obtain a current vector embedding;
maintain a vector database comprising previous vector embeddings of ECG signals of multiple patients acquired over time;
compare the current vector embedding with the previous vector embeddings; and
identify the patient based on one or more previous vector embeddings that are the closest to the current vector embedding.

11. A monitoring system comprising a cardiovascular monitor and the system of claim 10.
